# FASCICULE DE BREVET EUROPEEN

(11) **EP 0 950 051 B1**
(45) Date de publication et mention de la délivrance du brevet: **17.09.2003**
(21) Numéro de dépôt: 97952987.2
(22) Date de dépôt: 23.12.1997
(51) Int. Cl.: C07D 211/70, A61K 31/44

(54) **FORME MICROPARTICULAIRE D'UN DERIVE DE TETRAHYDROPYRIDINE**
MIKROPARTIKULARFORM EINES TETRAHYDROPYRIDINDERIVATES
MICRO-PARTICULATE FORM OF A TETRAHYDROPYRIDIN DERIVATIVE

(30) Priorité: 23.12.1996 FR 9615905
(43) Date de publication de la demande: 20.10.1999
(73) Titulaire: SANOFI-SYNTHELABO, 75013 Paris (FR)
(72) Inventeur: CARON, Antoine, F-34560 Montbazin (FR); CHAMBON, Jean-Pierre, F-34370 Prades le Lez (FR); MONNIER, Olivier, F-34560 Villeveyrac (FR)
(74) Mandataire: Gillard, Marie-Louise
(86) Numéro de dépôt international: FR9702394
(87) Numéro de publication internationale: WO98028272

(56) Documents cités:
- EP-A- 0 101 381

## Description

La présente invention concerne une forme microparticulaire du chlorhydrate de la 1-[2-(2-naphtyl)éthyl]-4-(3-trifluorométhylphényl)-1,2,3,6-tétrahydropyridine.

La 1-[2-(2-naphtyl)éthyl]-4-(3-trifluorométhylphényl)-1,2,3,6-tétrahydropyridine, ci-après désignée par son numéro de code SR 57746, et ses sels pharmaceutiquement acceptables ont été décrits pour la première fois dans EP 0 101 381 comme étant des agents anorexigènes et, par la suite, comme anti-anxiodépresseurs (US 5,026,716), anticonstipants (US 5,109,005), neurotrophiques (US 5,270,320), anti-radicaux libres (US 5,292,745) et cardioprotecteurs (US 5,378,709).

Dans EP 0 101 381, le SR 57746 est décrit sous forme de chlorhydrate, ci-après désigné SR 57746 A, et ce sel a été utilisé dans les essais précliniques et cliniques chez des volontaires sains (Phase I). Le SR 57746, selon ce document, est isolé par cristallisation dans l'éthanol, notamment dans l'éthanol absolu.

Dans les essais précliniques, notamment dans les tests de pharmacologie animale et de toxicologie, le SR 57746 a montré une activité et un comportement constants. De même, les études de pharmacocinétique chez les animaux ont donné des résultats constants et reproductibles.

Par contre, lors des études cliniques effectuées sur des volontaires sains, il a été constaté que le SR 57746 A administré par voie orale montre une grande variabilité dans les concentrations plasmatiques et dans les effets pharmacodynamiques du principe actif.

Dans les premiers essais cliniques chez des malades atteints de maladies très graves, notamment de sclérose latérale amyotrophique, la dose de SR 57746 A a été maintenue très basse, à savoir de 2 mg/jour et à cette dose le produit s'est montré prometteur (W. G. Bradley presentation on "New drugs for amyotrophic lateral sclerosis", American Academy of Neurology meeting, March 23-30, 1996; pages 240-23/240-28).

Il a été par ailleurs constaté que dans la préparation de plus grandes quantités de SR 57746 A selon le procédé d'isolement décrit dans EP 0 101 381 on ne réussit pas à obtenir un produit ayant des caractéristiques constantes permettant de pallier les inconvenients notés lors des études cliniques de Phase I.

Plus particulièrement, il a été constaté que selon le procédé d'isolement décrit dans EP 0 101 381 on obtient un SR 57746 A constitué par des cristaux dont la taille n'est pas constante et, notamment, est supérieure à 150 micromètres, plus particulièrement est de 150-600 micromètres dans au moins environ 75% des cristaux.

On a maintenant trouvé qu'en isolant le SR 57746 A par recristallisation dans de l'éthanol absolu sous agitation, on obtient un SR 57746 A formé par des cristaux ayant dans au moins 55% de la population une taille inférieure à 50 micromètres et que le produit ainsi obtenu possède une activité plus élevée lorsqu'il est administré en clinique humaine par voie orale.

Il a été également trouvé qu'en soumettant une solution de SR 57746 A dans de l'éthanol, contenant éventuellement de l'eau, à une opération d'atomisation, on obtient un principe actif sous forme essentiellement amorphe qui a un taux d'absorption constant chez l'homme et une activité très élevée permettant l'administration du principe actif à des dosages très faibles.

Il a été aussi trouvé que ladite atomisation donne de petites particules sphériques, dont le diamètre est inférieur à 15 micromètres, de façon constante et reproductible permettant de pallier les inconvenients dûs à la variabilité des caractéristiques du SR 57746 A isolé comme décrit dans EP 0 101 381.

Il a été enfin trouvé qu'un résultat identique est obtenu par micronisation du SR 57746 A obtenu par cristallisation dans l'éthanol absolu, comme décrit dans EP 0 101 381, de façon à obtenir des cristaux de taille inférieure à 50 micromètres.

Ainsi, selon un de ses aspects, la présente invention a pour objet une forme microparticulaire du chlorhydrate de 1-[2-(2-naphtyl)éthyl]-4-(3-trifluorométhylphényl)-1,2,3,6-tétrahydropyridine constitué par des microparticules, dont au moins 55% de la population a un diamètre inférieur à 50 micromètres.

Les microparticules selon la présente invention peuvent être des microsphères susceptibles d'être obtenues par atomisation ou des microcristaux obtenus par tamisage ou par micronisation.

L'expression "diamètre inférieur à 50 micromètres" se réfère aussi bien aux microsphères qu'aux microcristaux, ces derniers étant assimilables à des microsphères.

Avantageusement, la taille des microparticules selon la présente invention correspond à un diamètre inférieur à 25 micromètres, de préférence inférieur à 15 micromètres. Des microparticules ayant dans leur plus grande partie (80-85%) un diamètre inférieur à 10 micromètres sont particulièrement préférées.

Un SR 57746 A ayant une granulométrie fine, à savoir un produit formé d'une population de cristaux dont au moins 55% ont une taille inférieure à 50 micromètres peut être préparé par une recristallisation du produit obtenu selon EP 0 101 381 caractérisé en ce qu'on chauffe ledit produit dans de l'éthanol absolu sous agitation, on arrête le chauffage lorsque la dissolution est complète et l'agitation lorsque la température atteint environ 40°C, on laisse le mélange 16 à 60 heures à la température ambiante, puis on l'agite énergiquement à 10-18°C, on filtre et on sèche.

De façon alternative, un SR 57746A ayant la même granulométrie fine peut être obtenu en opérant comme décrit dans EP 0 101 381, en faisant réagir la 4-(3-trifluorométhylphényl)-1,2,3,6-tétrahydropyridine avec le 2-(2-chloroéthyl)naphtalène en présence de triéthylamine ou bien en soumettant la 1-(2-naphtylacétyl)-4-(3-trifluorométhylphényl)-1,2,3,6-tétrahydropyridine à une réduction avec l'hydrure de lithium et d'aluminium, mais en reprenant ensuite le résidu constitué par la 1-[2-(2-naphtyl)éthyl]-4-(3-trifluorométhylphényl)-1,2,3,6-tétrahydropyridine base directement avec de l'acide chlorhydrique dans de l'éthanol absolu à reflux et en opérant ensuite comme illustré ci-dessus.

Les microparticules selon la présente invention peuvent également être préparées par atomisation de solutions de SR 57746 A, avantageusement dans des (C₁-C₃)alcanols, dans des (C₃-C₆)alcanones ou dans de l'acétate d'éthyle éventuellement en présence d'eau, de préférence par atomisation d'une solution de SR 57746 A dans de l'éthanol contenant de 0 à 40% d'eau, dans un atomiseur classique par exemple un mini Spray Dryer Buchi, en règlant le débit de la pompe, l'aspiration, le chauffage, le flux de courant de façon à établir une température d'entrée entre 150 et 190°C, une température de sortie entre 50 et 120°C et une dépression de 30 à 70 mbar.

Par atomisation de ces solutions, on obtient des petites particules sphériques de taille inférieure à 50 micromètres, dont notamment 80-85% peuvent avoir un diamètre inférieur à 10 micromètres qui, à l'analyse calorimétrique différentielle (DSC, de l'anglais Differential Scanning Calorimetry) effectuée en utilisant un appareil DSC7 Perkin Elmer, calibré par référence à l'indium et au cyclohexane, présentent un monopic évasé de 130 à 160°C dont le maximum est à 146 ± 3°C.

Avantageusement les microparticules selon la présente invention sont préparées par micronisation du SR 57746 A obtenu comme décrit dans EP 0 101 381. Cette micronisation peut être effectuée dans un appareil classique permettant d'obtenir des microcristaux ayant une taille inférieure à 50 micromètres, par exemple dans un microniseur ALPINE 200 AS, en introduisant le SR 57746 A dans la chambre de micronisation (diamètre de 200 mm) à la vitesse de 15 à 50 kg/heure et à une pression de travail de 1 à 6,5 bar et en récupérant le produit dans une manche filtrante.

De façon particulièrement avantageuse, on opère dans des conditions permettant d'obtenir des microcristaux dont la population des particules a une taille moyenne inférieure à 25 micromètres, ou, encore mieux, inférieure à 15 micromètres. De préférence, on opère de manière à obtenir une population de microcristaux dont 80-85% ont une taille inférieure à 10 micromètres.

Si les microcristaux ainsi obtenus tendent à s'agréger, les agrégats peuvent être tamisés avant de préparer les compositions pharmaceutiques. L'agrégation éventuelle des microcristaux ne change cependant pas l'absorption du principe actif, comme il a été démontré dans le test des cellules CACO-2 illustré ci-dessous.

Pour éviter une telle agrégation, on peut éventuellement microniser le SR 57746 A en présence, par exemple, de mannitol, D-mannitol de préférence.

Comme indiqué ci-dessus, les microparticules selon la présente invention possèdent des propriétés qui les rendent particulièrement avantageuses pour la préparation des compositions pharmaceutiques les contenant.

Plus particulièrement, il a été démontré que la forme microcristalline non seulement permet de diminuer la quantité de dosage présente dans les compositions pharmaceutiques mais, surtout, permet de rendre uniforme l'absorption par voie orale et d'avoir ainsi une réponse thérapeutique constante de chaque patient. En outre, ladite absorption est indépendante des conditions de nourriture.

Une étude visant la détermination de l'absorption *in vitro* des microparticules selon la présente invention a été conduite en utilisant le modèle mono-couche CACO-2. Ce test, largement utilisé comme modèle épithélial intestinal prédictif pour l'absorption des médicaments (P. Artusson, Crit. Rev. Ther. Drug, 1991,8:305-330) a permis de mettre en évidence des différences significatives de dissolution et de perméabilité du SR 57746 A micronisé par rapport au SR 57746 A non micronisé ni atomisé.

Les résultats obtenus montrent que, dans le milieu utilisé (solution de Hank additionnée de sérum de veau foetal à 10% et d'acide taurocholique), les vitesses de dissolution et de perméabilité sont significativement différentes pour le SR 57746 A micronisé ou atomisé par rapport au SR 57746 A non micronisé ni atomisé. Plus particulièrement, il a été démontré que la dissolution et la perméabilité sont normalisées, à savoir rendues uniformes, après micronisation ou atomisation.

Les résultats obtenus *in vitro* ont été confirmés *in vivo* en comparant les observations obtenues dans deux études cliniques chez des volontaires sains: la première étude évaluant l'effet de la nourriture sur l'absorption par voie orale de SR 57746 A obtenu selon EP 0 101 381 et la seconde étude évaluant l'effet de la nourriture sur l'absorption par voie orale de SR 57746 A de l'Exemple 5 ci-dessous. Dans les deux études, le critère d'évaluation de l'absorption était l'aire sous la courbe des concentrations plasmatiques de SR 57746 en fonction du temps.

L'analyse des résultats a montré que:
- lorsque l'administration est effectuée avec le repas, il faut une dose de SR 57746 A préparée selon EP 0 101 381 trois à quatre fois supérieure à celle du produit de l'Exemple 5 ci-dessous pour obtenir la même absorption;
- lorsque l'administration est effectuée à jeun, il faut une dose de SR 57746 A préparée selon EP 0 101 381 environ neuf fois supérieure à celle du produit de l'Exemple 5 ci-dessous pour obtenir la même absorption.

Dans ces études il a été constaté, de façon surprenante, que dans le cas de l'administration du SR 57746 A préparé selon EP 0 101 381, l'absorption est deux à trois fois supérieure quand le produit est pris au repas alors que dans le cas de l'administration du produit de l'Exemple 5, l'absorption est la même que le produit soit administré à jeun ou avec la nourriture.

Ces résultats mettent en évidence l'intérêt de la présente invention qui permet de fournir un produit ayant une meilleure absorption qui n'est pas influencée par la prise de nourriture.

Ainsi, selon un autre de ses aspects, la présente invention concerne des compositions pharmaceutiques contenant, en tant que principe actif, une forme microparticulaire du chlorhydrate de l-[2-(2-naphtyl)éthyl]-4-(3-trifluorométhylphényl)-1,2,3,6-tétrahydropyridine constituée par des microparticules dont au moins 55% de la population a une taille inférieure à 50 micromètres, avantageusement inférieure à 25 micromètres, de préférence pour 80-85% des particules, inférieure à 10 micromètres.

La quantité de principe actif à administrer dépend de la nature et de la gravité des affections à traiter ainsi que du poids des malades. Néanmoins, la quantité de principe actif présent dans l'unité de dosage peut être de 0,1 à 5 mg, avantageusement de 0,5 à 3 mg, de préférence 2 mg (calculés en base libre). Les doses unitaires préférées comprendront généralement 0,5, 1, 1,5, 2, 2,5 ou 3 mg (calculés en base libre) de produit micronisé.

Ces doses unitaires seront administrées normalement une ou plusieurs fois par jour, par exemple une ou deux fois par jour, la dose globale chez l'homme étant variable entre 0,2 et 10 mg par jour, avantageusement entre 1 et 6 mg par jour (calculés en base libre).

Dans les compositions pharmaceutiques de la présente invention, le principe actif peut être administré sous formes unitaires d'administration en mélange avec des supports pharmaceutiques classiques, aux animaux et aux êtres humains pour le traitement des affections indiquées dans les brevets US 5 026 716, 5 109 005 , 5 270 320 , 5 292 745 , 5 378 709 , notamment pour le traitement de la neurodégénérescence. Les formes unitaires d'administration appropriées comprennent les comprimés éventuellement sécables, les gélules, les poudres, les granules.

Lorsqu'on prépare une composition solide sous forme de comprimés, on mélange le principe actif avec un véhicule pharmaceutique, tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Les compositions de l'invention peuvent être également préparées par un procédé d'extrusion-sphéronisation qui permet d'obtenir des sphéroïdes ayant la taille souhaitée. Par ce procédé, on mélange le SR 57746 A microparticulaire, de préférence atomisé ou micronisé, avec les excipients et avec de l'eau déminéralisée, on soumet la masse ainsi obtenue à une granulation et à une extrusion de façon à obtenir une masse d'extrusion coulant librement à travers des orifices ayant le diamètre souhaité, on sphéronise l'extrudat de façon à obtenir des sphéroïdes dont le diamètre est celui des orifices, on sèche les sphéroïdes ainsi obtenus et, de préférénce, on les introduit dans des gélules. De cette manière, le SR 57746 A et les excipients sont mélangés de façon à obtenir une composition pharmaceutique prête à être utilisée.

Les exemples suivants illustrent l'invention.

### EXEMPLE 1

En opérant dans les conditions décrites dans l'Exemple 1 de EP 0 101 381, on fait réagir la 4-(3-trifluorométhylphényl)-1,2,3,6-tétrahydropyridine, en présence de triéthylamine, avec le 2-(2-chloroéthyl)naphtalène dans de l'éthanol au reflux pendant 24 heures. Le mélange est concentré à sec, le résidu est repris dans l'éther éthylique et la solution éthérée, filtrée et lavée à l'eau, est séchée et évaporée.

On isole par la suite le chlorhydrate de 1-[2-(2-naphtyl)éthyl]-4-(3-trifluorométhylphényl)-1,2,3,6-tétrahydropyridine de la façon suivante: on reprend le résidu avec de l'acide chlorhydrique dans de l'éthanol 100 et on chauffe au reflux sous agitation. Lorsque la dissolution est complète, on arrête le chauffage et on laisse refroidir sous agitation. Après une dizaine de minutes, on arrête l'agitation et on laisse le mélange à la température ambiante pendant 48 heures. On filtre, on lave le précipité avec de l'éthanol absolu, on réempâte le gâteau dans de l'éthanol absolu avec agitation pneumatique, on filtre et on sèche à 40°C sous vide.

On obtient ainsi, avec un rendement global de 10%, un SR 57746 A dont la distribution particulaire est donnée dans le Tableau I.

**TABLEAU I**

| Taille en micromètres | Pourcentage |
|---|---|
| 4,0-6,0 | 0,8 |
| 6,0-8,0 | 2,6 |
| 8,0-10,0 | 3,8 |
| 10,0-14,0 | 6,3 |
| 14,0-20,0 | 6,4 |
| 20,0-30,0 | 13,9 |
| 30,0-40,0 | 15,8 |
| 40,0-50,0 | 9,6 |
| 50,0-60,0 | 4,9 |
| 60,0-70,0 | 3,4 |
| 70,0-80,0 | 1,8 |
| 80,0-90,0 | 1,9 |
| 90,0-100,0 | 1,8 |
| 100,0-150,0 | 8,1 |
| 150,0-200,0 | 6,2 |
| 200,0-300,0 | 7,5 |
| 300,0-400,0 | 3,6 |
| 400,0-500,0 | 1,6 |
| 500,0-600,0 | 0,1 |

La forme microparticulaire du SR 57746 A ainsi obtenue contient 59,2% de particules ayant une taille inférieure à 50 micromètres.

### EXEMPLE 2

Un mélange de 636 g de SR 57746 A, obtenu comme décrit dans EP 0 101 381 et formé de cristaux dont 77% a une taille entre 150 et 600 micromètres, dans 5 volumes d'éthanol absolu est chauffé au reflux sous agitation jusqu'à dissolution complète du produit, puis on arrête le chauffage et, lorsque la température atteint 40°C, on arrête l'agitation et on laisse le mélange 16 heures à la température ambiante. On porte le mélange à 16 °C sous agitation énergique et, après 10-20 minutes dans ces conditions, on filtre et on sèche sous vide à 40°C pendant 24 heures. On obtient ainsi 415 g de SR 57746 A constitué par une population de microparticules dont 60,3% a une taille inférieure à 50 micromètres.

### EXEMPLE 3

Une solution de 3 g de SR 57746 A dans 300 ml d'éthanol est atomisée dans un appareil "mini Spray Dryer Buchi" selon le principe de l'atomisation par buse en courant parallèle, en règlant le débit de la pompe, l'aspiration, le chauffage et le flux de courant de façon à avoir une température d'entrée de 172°C, une température de sortie de 107°C et une dépression de 40 mbar. Dans ces conditions, on obtient un produit monopic évasé en DSC avec le maximum à 145°C. Les particules obtenues sont sphériques et la population très homogène ne dépasse pas 5 micromètres de taille moyenne.

### EXEMPLE 4

Une solution de 3 g de SR 57746 A dans 210 ml d'éthanol et dans 90 ml d'eau est atomisée dans un appareil décrit dans l'Exemple 3, selon le principe de l'atomisation par buse en courant parallèle, en règlant le débit de la pompe, l'aspiration, le chauffage et le flux de courant de façon à avoir une température d'entrée de 172°C, une température de sortie de 63°C et une dépression de 60 mbar. Dans ces conditions, on obtient un SR 57746 A atomisé, essentiellement amorphe qui, au thermogramme DSC a montré un seul pic évasé avec un maximum à 147,6°C. Les particules obtenues sont sphériques et la population très homogène ne dépasse pas 5 micromètres de taille moyenne.

### EXEMPLE 5

On introduit dans la chambre de micronisation (diamètre 200 mm) d'un microniseur ALPINE 200 AS 24 kg de SR 57746 A à une vitesse de 25 kg/heure et à une pression de travail de 6,5 bar et on récupère le produit ainsi micronisé dans une manche filtrante. On obtient ainsi un SR 57746 A micronisé ayant une distribution particulaire selon laquelle la totalité des particules a une taille inférieure à 20 micromètres et 85% des particules ont une taille inférieure à 10 micromètres.

### EXEMPLE 6

Composition pharmaceutique contenant, en tant que principe actif, le SR 57746 A micronisé selon l'Exemple 5 ci-dessus:

| | |
|---|---|
| Principe actif | 2,192 mg |
| Amidon de maïs | 141,218 mg |
| Silice colloïdale anhydre | 0,200 mg |
| Stéarate de magnésium | 0,400 mg |

Le principe actif est tamisé à 0,2 mm, puis prémélangé avec les excipients. Ce mélange est tamisé à 0,315 mm, remélangé, puis à nouveau tamisé à 0,315 mm. Après un dernier mélange, on introduit la composition dans des gélules de gélatine n. 3, à raison de 170 mg de composition contenant une quantité de SR 57746 A micronisé correspondant à 2 mg de 1-[2-(2-naphtyl)éthyl]-4-(3-trifluorométhylphényl)-1,2,3,6-tétrahydropyridine base.

## Revendications

1. Forme microparticulaire du chlorhydrate de 1-[2-(2-naphtyl)éthyl]-4-(3-trifluorométhylphényl)-1,2,3,6-tétrahydropyridine constitué par des particules dont au moins 55% de la population a un diamètre inférieur à 50 micromètres.

2. Forme microparticulaire selon la revendication 1, **caractérisée en ce que** le diamètre des particules est inférieur à 25 micromètres.

3. Forme microparticulaire selon la revendication 2, **caractérisée en ce que** le diamètre des particules est inférieur à 15 micromètres.

4. Forme microparticulaire selon la revendication 3, **caractérisée en ce que** le diamètre de 80-85% de la population des particules est inférieur à 10 micromètres.

5. Forme microparticulaire selon l'une des revendications 1 à 4, **caractérisée en ce que** les particules sont des microsphères.

6. Forme microparticulaire selon la revendication 5, **caractérisée en ce que** les microparticules sont constituées par du chlorhydrate de 1-[2-(2-naphtyl)éthyl]-4-(3-trifluorométhylphényl)-1,2,3,6-tétrahydropyridine sous forme essentiellement amorphe.

7. Forme microparticulaire selon l'une des revendications 1 à 4, **caractérisée en ce que** les particules sont des cristaux micronisés.

8. Composition pharmaceutique contenant, en tant que principe actif, une forme microparticulaire selon l'une des revendications 1 à 7.

9. Composition selon la revendication 8, **caractérisée en ce qu'**elle est sous forme d'unité de dosage.

10. Composition selon la revendication 9, **caractérisée en ce que** chaque unité de dosage contient de 0,1 à 5 mg de principe actif (calculés en base libre).

11. Composition selon la revendication 10, **caractérisée en ce que** chaque unité de dosage contient de 0,5 à 3 mg de principe actif (calculés en base libre).

12. Composition selon la revendication 11, **caractérisée en ce que** chaque unité de dosage contient 2 mg de principe actif (calculés en base libre).

## Patentansprüche

1. Mikropartikulare Form von 1-[2-(2-Naphthyl)ethyl]-4-(3-trifluormethylphenyl)-1,2,3,6-tetrahydropyridin-Hydrochlorid bestehend aus Partikeln, wobei mindestens 55 % der Population der Partikel einen Durchmesser unter 50 µm aufweist.

2. Mikropartikulare Form nach Anspruch 1, **dadurch gekennzeichnet, dass** der Durchmesser der Partikel unter 25 µm liegt.

3. Mikropartikulare Form nach Anspruch 2, **dadurch gekennzeichnet, dass** der Durchmesser der Partikel unter 15 µm liegt.

4. Mikropartikulare Form nach Anspruch 3, **dadurch gekennzeichnet, dass** der Durchmesser von 80 bis 85 % der Partikelpopulation unter 10 µm liegt.

5. Mikropartikulare Form nach einem der Ansprüche 1 bis 4, **dadurch gekennzeichnet, dass** es sich bei den Partikeln um Mikrokugeln handelt.

6. Mikropartikulare Form nach Anspruch 5, **dadurch gekennzeichnet, dass** die Mikropartikel aus 1-[2-(2-Naphthyl)ethyl]-4-(3-trifluormethylphenyl)-1,2,3,6-tetrahydropyridin-Hydrochlorid in im Wesentlichem amorpher Form bestehen.

7. Mikropartikulare Form nach einem der Ansprüche 1 bis 4, **dadurch gekennzeichnet, dass** die Partikel mikronisierte Kristalle sind.

8. Pharmazeutische Zusammensetzung, die als Wirkstoff eine mikropartikulare Form nach einem der Ansprüche 1 bis 7 enthält.

9. Zusammensetzung nach Anspruch 8, **dadurch gekennzeichnet, dass** sie als Dosiereinheit vorliegt.

10. Zusammensetzung nach Anspruch 9, **dadurch gekennzeichnet, dass** jede Dosiereinheit 0,1 bis 5 mg Wirkstoff (als freie Base berechnet) enthält.

11. Zusammensetzung nach Anspruch 10, **dadurch gekennzeichnet, dass** jede Dosiereinheit 0,5 bis 3 mg Wirkstoff (als freie Base berechnet) enthält.

12. Zusammensetzung nach Anspruch 11, **dadurch gekennzeichnet, dass** jede Dosiereinheit 2 mg Wirkstoff (als freie Base berechnet) enthält.

## Claims

1. A microparticulate form of 1-[2-(naphth-2-yl)ethyl]-4-(3-trifluoromethylphenyl)-1,2,3,6-tetrahydropyridine hydrochloride consisting of particles for which at least 55% of the population have a diameter below 50 micrometers.

2. A microparticulate form according to claim 1 **characterized in that** the particle diameter is below 25 micrometers.

3. A microparticulate form according to claim 2 **characterized in that** the particle diameter is below 15 micrometers.

4. A microparticulate form according to claim 3 **characterized in that** the diameter of 80 - 85% of the population of particles is below 10 micrometers.

5. A microparticulate form according to one of claims 1 to 4 **characterized in that** the particles are microspheres.

6. A microparticulate form according to claim 5 **characterized in that** the microparticles consist of 1-[2-(naphth-2-yl)ethyl]-4-(3-trifluoromethylphenyl)-1,2,3,6-tetrahydropyridine hydrochloride in an essentially amorphous form.

7. A microparticulate form according to one of claims 1 to 4 **characterized in that** the particles are micronized crystals.

8. A pharmaceutical composition containing a microparticulate form according to one of claims 1 to 7 as the active principle.

9. A composition according to claim 8 **characterized in that** it is in the form of a dosage unit.

10. A composition according to claim 9 **characterized in that** each dosage unit contains from 0.1 to 5 mg of active principle (calculated as the free base).

11. A composition according to claim 10 **characterized in that** each dosage unit contains from 0.5 to 3 mg of active principle (calculated as the free base).

12. A composition according to claim 11 **characterized in that** each dosage unit contains 2 mg of active principle (calculated as the free base).
